# EUROPEAN PATENT APPLICATION

(11) **EP 4 092 084 A1**
(43) Date of publication of application: **23.11.2022**
(21) Application number: 21757917.6
(22) Date of filing: 02.02.2021
(51) Int. Cl.: C08L 101/00, G06N 20/00, G16C 20/30, G16C 20/70

(54) **INFORMATION PROCESSING SYSTEM, INFORMATION PROCESSING METHOD, AND INFORMATION PROCESSING PROGRAM**

(30) Priority: 18.02.2020 JP 2020025017
(71) Applicant: Showa Denko Materials Co., Ltd., Tokyo 100-6606 (JP)
(72) Inventor: HANAOKA Kyohei, Tokyo 100-6606 (JP)
(74) Representative: Berggren Oy
(86) International application number: PCT/JP2021/003767
(87) International publication number: WO 2021/166634

(57) **Abstract**

An information processing system according to an embodiment is configured to: acquire a numerical representation and a combination ratio for each of a plurality of component objects; execute, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and output the composite feature vector.

## Description

### Technical Field

One aspect of the present disclosure relates to an information processing system, an information processing method, and an information processing program.

### Background Art

A method of analyzing a composite object obtained by combining a plurality of component objects using machine learning has been used. For example, Patent Literature 1 describes a method of predicting the bondability between the three-dimensional structure of a biopolymer and the three-dimensional structure of a compound. This method includes: generating a predicted three-dimensional structure of a complex of a biopolymer and a compound based on the three-dimensional structure of the biopolymer and the three-dimensional structure of the compound; converting the predicted three-dimensional structure into a predicted three-dimensional structure vector representing a result of comparison with an interaction pattern; and predicting the bondability between the three-dimensional structure of the biopolymer and the three-dimensional structure of the compound by determining the predicted three-dimensional structure vector using a machine learning algorithm.

### Citation List

### Patent Literature

Patent Literature 1: JP 2019-28879 A

### Summary of Invention

### Technical Problem

When there are various or many component objects, it is not possible to prepare a sufficient amount of data for these component objects. As a result, the accuracy of analysis of a composite object may not reach the expected level. Therefore, there has been a demand for a mechanism for improving the accuracy of analysis of a composite object even in a case where a sufficient amount of data cannot be prepared for component objects.

### Solution to Problem

An information processing system according to one aspect of the present disclosure includes at least one processor. The at least one processor is configured to: acquire a numerical representation and a combination ratio for each of a plurality of component objects; execute, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and output the composite feature vector.

An information processing method according to one aspect of the present disclosure is executed by an information processing system including at least one processor. The information processing method includes: acquiring a numerical representation and a combination ratio for each of a plurality of component objects; executing, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and outputting the composite feature vector.

An information processing program according to one aspect of the present disclosure causes a computer to execute: acquiring a numerical representation and a combination ratio for each of a plurality of component objects; executing, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and outputting the composite feature vector.

In such an aspect, since the machine learning and the application of the combination ratios are executed for each component object, it is possible to improve the accuracy of analysis of composite object even in a case where a sufficient amount of data cannot be prepared for the component objects.

### Advantageous Effects of Invention

According to one aspect of the present disclosure, it is possible to improve the accuracy of analysis of composite object even in a case where a sufficient amount of data cannot be prepared for the component objects.

### Brief Description of Drawings

FIG. 1 is a diagram showing an example of the hardware configuration of a computer configuring an information processing system according to an embodiment.
FIG. 2 is a diagram showing an example of the functional configuration of the information processing system according to the embodiment.
FIG. 3 is a flowchart showing an example of an operation of the information processing system according to the embodiment.
FIG. 4 is a diagram showing an example of a procedure for calculating a composite feature vector.
FIG. 5 is a diagram showing an example of applying combination ratios in the middle of machine learning.
FIG. 6 is a diagram showing a specific example of a procedure for calculating a composite feature vector.
FIGS. 7A, 7B and 7C are diagrams showing another example of a procedure for calculating a composite feature vector.

### Description of Embodiments

Hereinafter, an embodiment in the present disclosure will be described in detail with reference to the accompanying diagrams. In addition, in the description of the diagrams, the same or equivalent elements are denoted by the same reference numerals, and the repeated description thereof will be omitted.

### [System overview]

An information processing system 10 according to the embodiment is a computer system that performs an analysis on a composite object obtained by combining a plurality of component objects at a predetermined combination ratio. A component object refers to a tangible object or an intangible object used to generate a composite object. The composite object can be a tangible object or an intangible object. Examples of a tangible object include any substance or object. Examples of an intangible object include data and information. "Combining a plurality of component objects" refers to a process of making a plurality of component objects into one object, that is, a composite object. The method of combining is not limited, and may be, for example, compounding, blending, synthesis, bonding, mixing, merging, combination, chemical combination, or uniting, or other methods. The analysis of a composite object refers to a process for obtaining data indicating a certain feature of the composite object.

The plurality of component objects may be any plurality of types of materials. In this case, the composite object is a multi-component substance produced by these materials. The materials are arbitrary components used to produce a multi-component substance. For example, the plurality of materials may be any plurality of types of molecules, atoms, molecular structures, crystal structures, or amino acid sequences. In this case, the composite object is a multi-component substance obtained by combining those molecules, atoms, molecular structures, crystal structures, or amino acid sequences using an arbitrary method. For example, the material may be a polymer and correspondingly the multi-component substance may be a polymer alloy. The material may be a monomer and correspondingly the multi-component substance may be a polymer. The material may be a medicinal substance, that is, a chemical substance having a pharmacological action, and correspondingly, the multi-component substance may be a medicine.

The information processing system 10 performs machine learning for the analysis of a composite object. The machine learning is a method of autonomously finding a law or rule by learning based on given information. The specific method of machine learning is not limited. For example, the information processing system 10 may perform machine learning using a machine learning model that is a calculation model configured to include a neural network. The neural network is an information processing model that imitates the mechanism of the human cranial nerve system. As a more specific example, the information processing system 10 may perform machine learning by using at least one of graph neural network (GNN), convolutional neural network (CNN), recurrent neural network (RNN), attention RNN, and multi-head attention.

### [System configuration]

The information processing system 10 is configured to include one or more computers. In a case where a plurality of computers are used, one information processing system 10 is logically constructed by connecting these computers to each other through a communication network, such as the Internet or an intranet.

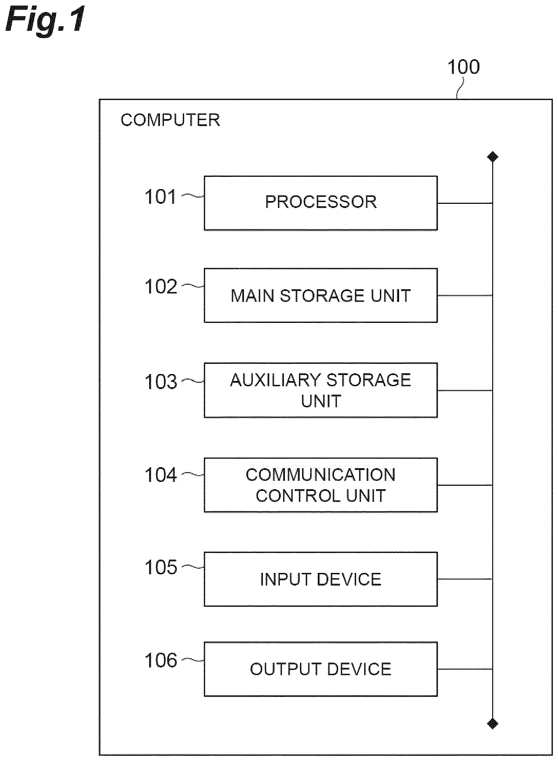

FIG. 1 is a diagram showing an example of a general hardware configuration of a computer 100 configuring the information processing system 10. For example, the computer 100 includes a processor (for example, a CPU) 101 for executing an operating system, an application program, and the like, a main storage unit 102 configured by a ROM and a RAM, an auxiliary storage unit 103 configured by a hard disk, a flash memory, and the like, a communication control unit 104 configured by a network card or a wireless communication module, an input device 105 such as a keyboard and a mouse, and an output device 106 such as a monitor.

Each functional element of the information processing system 10 is realized by reading a predetermined program on the processor 101 or the main storage unit 102 and causing the processor 101 to execute the program. The processor 101 operates the communication control unit 104, the input device 105, or the output device 106 according to the program and performs reading and writing of data in the main storage unit 102 or the auxiliary storage unit 103. The data or database required for the processing is stored in the main storage unit 102 or the auxiliary storage unit 103.

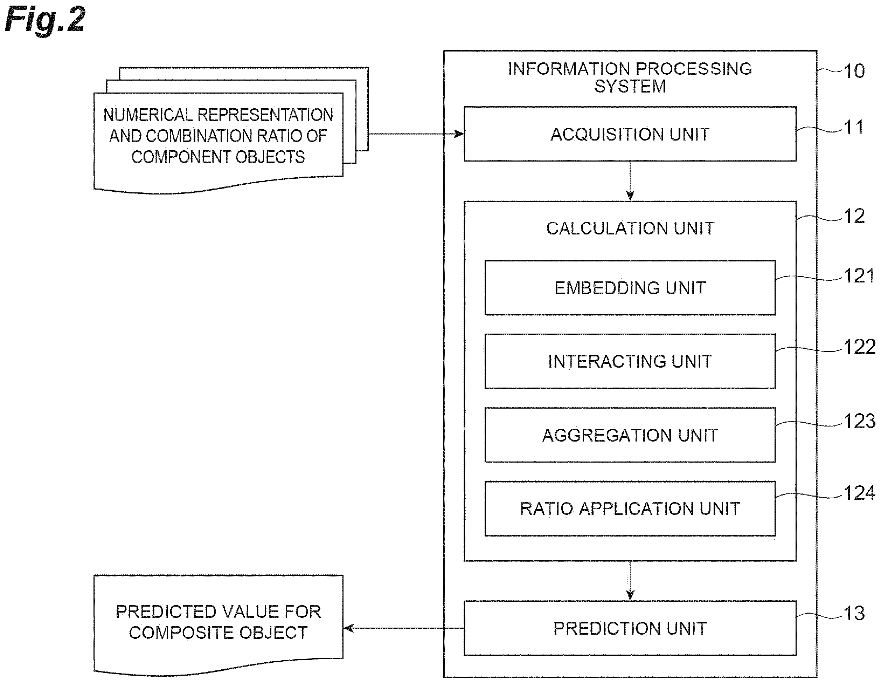

FIG. 2 is a diagram showing an example of the functional configuration of the information processing system 10. The information processing system 10 includes an acquisition unit 11, a calculation unit 12, and a prediction unit 13 as functional elements.

The acquisition unit 11 is a functional element for acquiring data relevant to a plurality of component objects. Specifically, the acquisition unit 11 acquires a numerical representation and a combination ratio for each of the plurality of component objects. The numerical representation of a component object refers to data representing arbitrary attributes of the component object using a plurality of numerical values. The attributes of the component object refer to the properties or features of the component object. The numerical representation may be visualized by various methods. For example, the numerical representation may be visualized by methods such as numbers, letters, texts, molecular graphs, vectors, images, time-series data, and the like or may be visualized by any combination of two or more of these methods. Each numerical value that makes up the numerical representation may be represented in decimal or may be represented in other notations such as a binary notation and a hexadecimal notation. The combination ratio of component objects refers to a ratio between a plurality of component objects. The specific type, unit, and representation method of the combination ratio are not limited, and may be arbitrarily determined depending on the component object or the composite object. For example, the combination ratio may be represented by a ratio such as a percentage or by a histogram, or may be represented by an absolute amount of each component object.

The calculation unit 12 is a functional element that executes, based on a plurality of the numerical representations and a plurality of the combination ratios corresponding to the plurality of component objects, the machine learning and the application of the plurality of combination ratios to calculate a composite feature vector. The composite feature vector refers to a vector indicating features of the composite object. The features of the composite object refers to any element that makes the composite object different from other objects. The vector is an n-dimensional quantity having n numerical values, and may be expressed as a one-dimensional array. In one example, the calculation unit 12 calculates a feature vector of each of the plurality of component objects in the process of calculating the composite feature vector. The feature vector is a vector indicating features of the component object. The features of the component object refers to any element that makes the component object different from other objects.

The calculation unit 12 includes an embedding unit 121, an interacting unit 122, an aggregation unit 123, and a ratio application unit 124. The embedding unit 121 is a functional element that generates, from a set of vectors, a different set of the same number of vectors using the machine learning. In one example, the embedder 121 generates the feature vector from unstructured data. The unstructured data is data that cannot be represented by a fixed-length vector. The interacting unit 122 is a functional element that uses the machine learning or other methods to generate, from a set of vectors, another set of the same number of vectors. In one example, the interacting unit 122 may receive an input of the feature vector already obtained by the machine learning. The aggregation unit 123 is a functional element that aggregates a vector set (a plurality of vectors) into one vector using the machine learning or other methods. The ratio application unit 124 is a functional element that applies the combination ratio.

The prediction unit 13 is a functional element that predicts characteristics of the composite object and outputs the predicted value. The characteristics of the composite object refer to unique properties of the composite object.

In one example, each of at least one machine learning model used in the present embodiment are trained models that are expected to have the highest estimation accuracy, and therefore can be referred to as "best machine learning models". However, it should be noted that the trained model is not always "best in reality". The trained model is generated by processing training data including many combinations of input vectors and labels with a given computer. The given computer calculates an output vector by inputting the input vector into the machine learning model, and obtains an error between a predicted value obtained from the calculated output vector and a label indicated by training data (that is, a difference between the estimation result and the ground truth). Then, the computer updates a predetermined parameter in the machine learning model based on the error. The computer generates a trained model by repeating such learning. The computer that generates a trained model is not limited, and may be, for example, the information processing system 10 or another computer system. The process of generating the trained model can be referred to as a learning phase, and the process of using the trained model can be referred to as an operation phase.

In one example, at least part of the machine learning model used in the present embodiment may be described by a function that does not depend on the order of inputs. This mechanism makes it possible to eliminate the influence of the order of the plurality of vectors in the machine learning.

### [Data]

As described above, each component object may be a material, and the composite object may be a multi-component substance. In this case, the numerical representation of the component object (material) may include a numerical value indicating the chemical structure of the material, or may include a numerical value indicating a configuration repetition unit (CRU) of the chemical structure of the material. The combination ratio may be a compounding ratio or a mixing ratio. The predicted value of the characteristics of the composite object (multi-component substance) may indicate at least one of the glass transition temperature (Tg) and elastic modulus of the multi-component substance.

### [Operation of system]

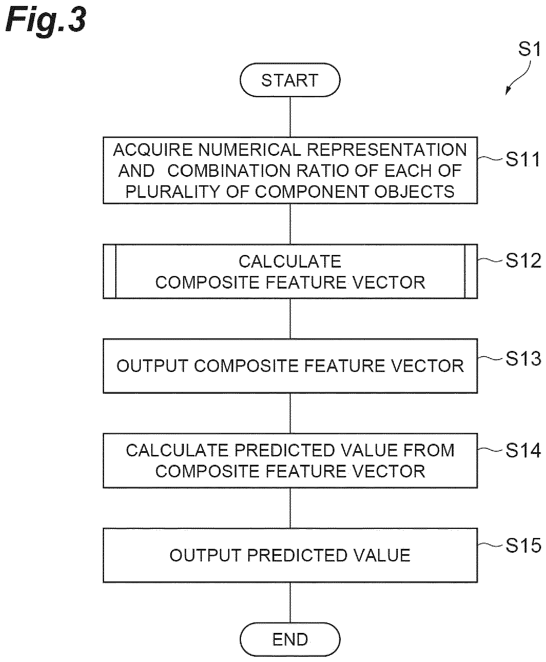

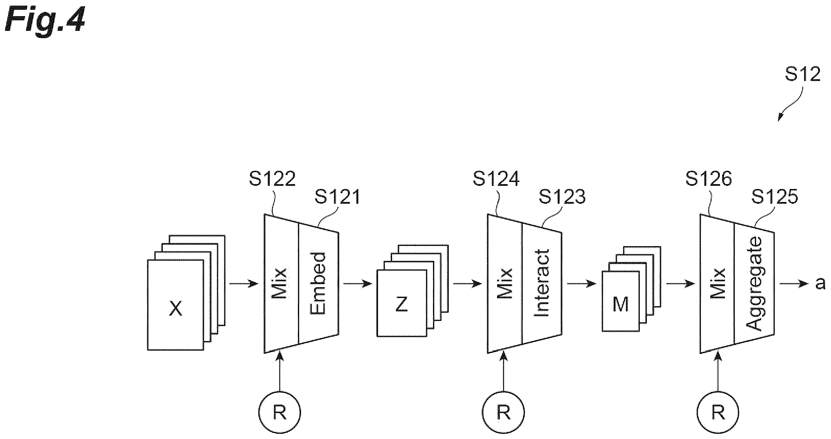

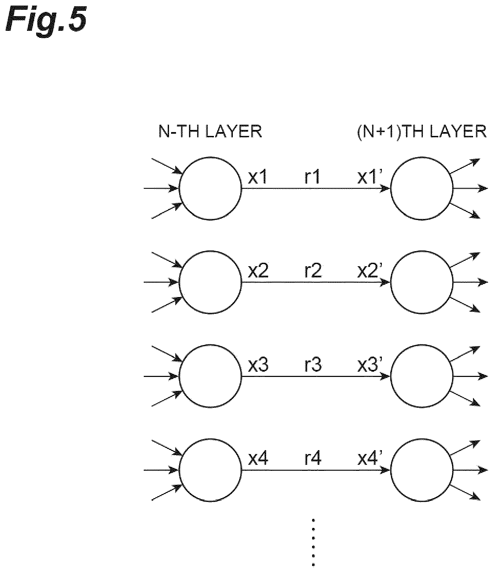

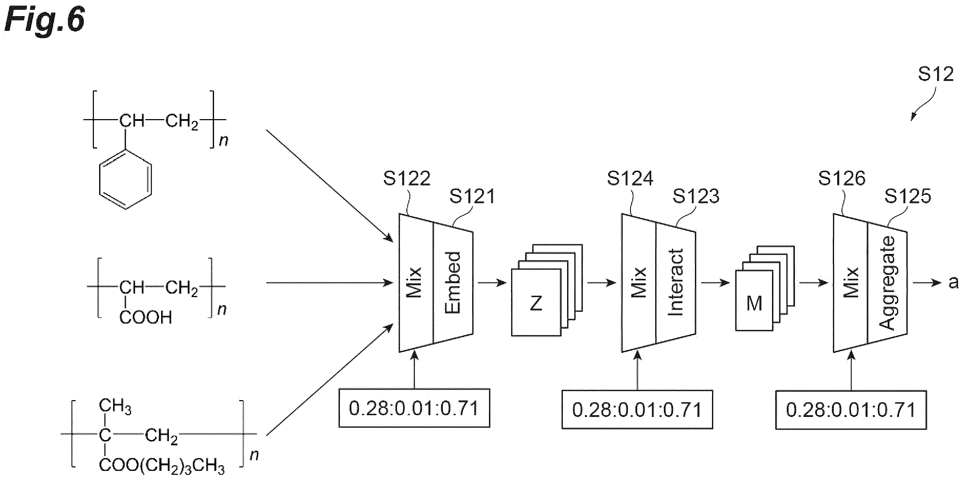

The operation of the information processing system 10 and the information processing method according to the present embodiment will be described with reference to FIGS. 3 to 6. FIG. 3 is a flowchart showing an example of the operation of the information processing system 10 as a processing flow S1. FIG. 4 is a diagram showing an example of a procedure for calculating the composite feature vector. FIG. 5 is a diagram showing an example of applying the combination ratios in the middle of the machine learning. FIG. 6 is a diagram showing a specific example of a procedure for calculating the composite feature vector, and corresponds to FIG. 4.

In step S11, the acquisition unit 11 acquires a numerical representation and a combination ratio for each of a plurality of component objects. Assuming that information on two component objects Ea and Eb is input, the acquisition unit 11 acquires, for example, a numerical representation {1, 1, 2, 3, 4, 3, 3, 5, 6, 7, 5, 4} of the component object Ea, a numerical representation {1, 1, 5, 6, 4, 3, 3, 5, 1, 7, 0, 0} of the component object Eb, and combination ratios {0.7, 0.3} of the component objects Ea and Eb. In this example, each numerical representation is shown as a vector. The combination ratios {0.7, 0.3} mean that the component objects Ea and Eb are used in a ratio of 7:3 to obtain a composite object.

The acquisition unit 11 may acquire the data of each of the plurality of component objects by using any method. For example, the acquisition unit 11 may read data by accessing a given database, or may receive data from another computer or computer system, or may receive data input by the user of the information processing system 10. Alternatively, the acquisition unit 11 may acquire data by any two or more of these methods.

In step S12, the calculation unit 12 calculates the composite feature vector based on the plurality of numerical representations and the plurality of combination ratios corresponding to the plurality of component objects. In this calculation, the calculation unit 12 executes each of the machine learning and the application of the combination ratios at least once. The procedure for calculating the composite feature vector is not limited, and various methods may be adopted.

An example of the details of step S12 will be described with reference to FIG. 4. In this example, the calculation unit 12 calculates a composite feature vector a based on a plurality of numerical representations X and a plurality of combination ratios R corresponding to a plurality of component objects.

In step S121, the embedding unit 121 calculates a feature vector Z from the numerical representation X for the plurality of component objects by machine learning for an embedding function for calculating features of vectors. In the embedding function, the input vector (the numerical representation X in this example) and the output vector (the feature vector Z in this example) are in a one to-one relationship. The embedding unit 121 inputs the plurality of numerical representations X corresponding to the plurality of component objects into a machine learning model for the embedding function to calculate the feature vector Z of each of the plurality of component objects. In one example, for each of the plurality of component objects, the embedding unit 121 inputs the numerical representation X corresponding to the component object into the machine learning model for the embedding function to calculate the feature vector Z of the component object. The feature vector Z refers to a vector indicating features of the component object. In one example, the machine learning model for the embedding function may generate the feature vector Z that is a fixed-length vector from the numerical representation X that is unstructured data. The machine learning model is not limited, and may be determined by any policy in consideration of factors such as types of component objects and composite object. For example, the embedding unit 121 may execute the machine learning for the embedding function using a graph neural network (GNN), a convolutional neural network (CNN), or a recurrent neural network (RNN).

In step S122, the ratio application unit 124 executes application of the combination ratio R in association with the embedding function (more specifically, the machine learning model for the embedding function). The timing of applying the combination ratio R is not limited. For example, the ratio application unit 124 may apply the combination ratios R to the numerical representations X, and thus step S122 may be executed prior to step S121. Alternatively, the ratio application unit 124 may apply the combination ratio R to the feature vector Z, and thus step S122 may be executed after step S121. Alternatively, the ratio application unit 124 may apply the combination ratio R to output data of a certain intermediate layer (that is, an intermediate result of the machine learning) in the middle of the machine learning for the embedding function, and thus step S122 may be a part of step S121.

In the present disclosure, "executing the application of a combination ratio in association with a certain function" refers to applying a combination ratio to at least one of input data of the function, output data of the function, or an intermediate result (intermediate data) of the function. "Executing the application of a combination ratio in association with a certain machine learning model" refers to applying a combination ratio to at least one of input data (input vector) to the machine learning model, output data (output vector) from the machine learning model, or an intermediate result (output data in a certain intermediate layer) in the machine learning model. In the present disclosure, "Applying a combination ratio (to certain target data)" refers to a process of changing the target data by the combination ratio. The method of applying the combination ratio is not limited. For example, the ratio application unit 124 may apply the combination ratio by connecting the combination ratio as an additional component to the target data. Alternatively, the ratio application unit 124 may execute the application by multiplying or adding the combination ratio to each component of the target data. The ratio application unit 124 may apply the combination ratio with such simple operations.

With reference to FIG. 5, an example processing for applying combination ratios in the middle of the machine learning having a multilayer structure will be described. In this example, the ratio application unit 124 applies a ratio to each of the output values from the N-th layer of the machine learning model. The output value to which the ratio is applied is processed as an input value of the (N+1)th layer. For each of the N-th and (N+1)th layers, each node indicates one corresponding component object. It is assumed that the output values of the nodes in the N-th layer are x1, x2, x3, x4, ..., and the combination ratios R of the plurality of component objects is represented by "r1 : r2 : r3 : r4 : ...". The combination ratios r1, r2, r3, and r4 correspond to the output values x1, x2, x3, and x4, respectively. The ratio application unit 124 calculates an output value x1' by applying the combination ratio r1 to the output value x1, calculates an output value x2' by applying the combination ratio r2 to the output value x2, calculates an output value x3' by applying the combination ratio r3 to the output value x3, and calculates an output value x4' by applying the combination ratio r4 to the output value x4. These output values x1', x2', x3' and x4' are processed as input values of the (N+1)th layer.

By applying the plurality of combination ratios to the output data of the intermediate layer of the machine learning model as in the example of FIG. 5, the combination ratios can be appropriately applied regardless of whether the data input to the machine learning model is unstructured or structured. As one example, it is assumed that a machine learning model is used that includes an embedding function for converting unstructured data into a fixed-length vector. In a case where the combination ratios are applied to the unstructured data before the processing by the machine learning model, the application is difficult because the correspondence between the individual numerical values of the unstructured data and the individual combination ratios is not obvious. In addition, the embedding function may not be able to perform its original performance on the unstructured data. In one example, by applying the plurality of combination ratios to output data of an intermediate layer of the machine learning model including the embedded function, it can be expected that the machine learning model delivers the desired performance.

In step S123, the interacting unit 122 calculates a different feature vector M from the feature vector Z for the plurality of component objects by machine learning for an interaction function for interacting the plurality of vectors. In the interaction function, the input vector (feature vector Z in this example) and the output vector (the different feature vector M in this example) are in a one to-one relationship. In one example, the interacting unit 122 inputs a set of the feature vectors Z corresponding to the plurality of component objects into the machine learning model for the interaction function to calculate the different feature vector M for each of the plurality of component objects. The machine learning model is not limited, and may be determined by any policy in consideration of factors such as types of component objects and composite object. For example, the interacting unit 122 may execute the machine learning for the interaction function using a convolutional neural network (CNN) or a recurrent neural network (RNN). In another example, the interacting unit 122 may calculate the feature vector M by an interaction function that does not use the machine learning.

In step S124, the ratio application unit 124 executes the application of the combination ratio R in association with the interaction function (more specifically, the machine learning model for the interaction function). The timing of applying the combination ratio R is not limited. For example, the ratio application unit 124 may apply the combination ratio R to the feature vector Z, and thus step S124 may be executed prior to step S123. Alternatively, the ratio application unit 124 may apply the combination ratio R to the different feature vector M, and thus step S124 may be executed after step S123. Alternatively, the ratio application unit 124 may apply the combination ratio R to output data of a certain intermediate layer (that is, an intermediate result of the machine learning) in the middle of machine learning for the interaction function, and thus step S124 may be a part of step S123. As described above, the method of applying the combination ratio is not limited.

In step S125, the aggregation unit 123 aggregates the plurality of vectors into one vector. In one example, the aggregation unit 123 calculates one composite feature vector a from the plurality of feature vectors M by machine learning for an aggregation function for aggregating the plurality of vectors into one vector. In the aggregation function, the input vector (the feature vector M in this example) and the output vector (composite feature vector a) are in an N: 1 relationship. In one example, the aggregation unit 123 inputs a set of the feature vectors M corresponding to the plurality of component objects into a machine learning model for the aggregation function to calculate the composite feature vector a. The machine learning model is not limited, and may be determined by any policy in consideration of factors such as types of component objects and composite objects. For example, the aggregation unit 123 may execute the machine learning for the aggregation function using a convolutional neural network (CNN) or a recurrent neural network (RNN). In another example, the aggregation unit 123 may calculate the composite feature vector a by an aggregation function that does not use the machine learning, and may calculate the composite feature vector a by adding the plurality of feature vectors M, for example.

In step S126, the ratio application unit 124 executes the application of the combination ratio R in association with the aggregation function. The timing of applying the combination ratio R is not limited. For example, the ratio application unit 124 may apply the combination ratio R to the feature vector M, and thus step S126 may be executed prior to step S125. Alternatively, the ratio application unit 124 may apply the combination ratio R to output data of a certain intermediate layer (that is, an intermediate result in the machine learning model) in the middle of the machine learning for the aggregation function. Therefore, step S126 may be a part of step S125. As described above, the method of applying the combination ratio is not limited.

In the example of FIG. 4, the feature vector Z is an example of a first feature vector. The feature vector M is an example of a second feature vector, and is also an example of a second feature vector reflecting the plurality of combination ratios. The machine learning model for the embedding function is an example of a first machine learning model, and the machine learning model for the interaction function is an example of a second machine learning model.

A specific example of step S12 will be described with reference to FIG. 6. In this example, three types of materials (polymers) of polystyrene, polyacrylic acid, and butyl polymethacrylate are shown as component objects. For each of these materials, a numerical representation X is provided in any form. The combination ratios in this example are 0.28 for polystyrene, 0.01 for polyacrylic acid and 0.71 for butyl polymethacrylate. The calculation unit 12 executes step S12 (more specifically, steps S121 to S126) based on these pieces of data to calculate a composite feature vector a indicating features of multi-component substance (polymer alloy) obtained from these three types of materials.

Returning to FIG. 3, in step S13, the calculation unit 12 outputs the composite feature vector. In the present embodiment, the calculation unit 12 outputs the composite feature vector to the prediction unit 13 for subsequent processing in the information processing system 10. However, the output method of the composite feature vector is not limited thereto, and may be designed in any policy. For example, the calculation unit 12 may store the composite feature vector in a given database, may transmit the composite feature vector to another computer or computer system, or may display the composite feature vector on a display device.

In step S14, the prediction unit 13 calculates a predicted value of characteristics of the composite object from the composite feature vector. A prediction method is not limited and may be designed in any policy. For example, the prediction unit 13 may calculate the predicted value from the composite feature vector by machine learning. Specifically, the prediction unit 13 inputs the composite feature vector into a given machine learning model to calculate the prediction value. The machine learning model for obtaining the predicted value is not limited, and may be determined by any policy in consideration of factors such as the type of the composite object. For example, the prediction unit 13 may execute the machine learning using any neural network that solves a regression problem or a classification problem. Typically, the predicted value of the regression problem is represented by a numerical value, and the predicted value of the classification problem indicates a category. The prediction unit 13 may calculate the predicted value using a method other than the machine learning.

In step S15, the prediction unit 13 outputs the prediction value. A method of outputting the predicted value is not limited. For example, the prediction unit 13 may store the prediction value in a given database, may transmit the prediction value to another computer or a computer system, or may display the prediction value on a display device. Alternatively, the prediction unit 13 may output the predicted value to another functional element for subsequent processing in the information processing system 10.

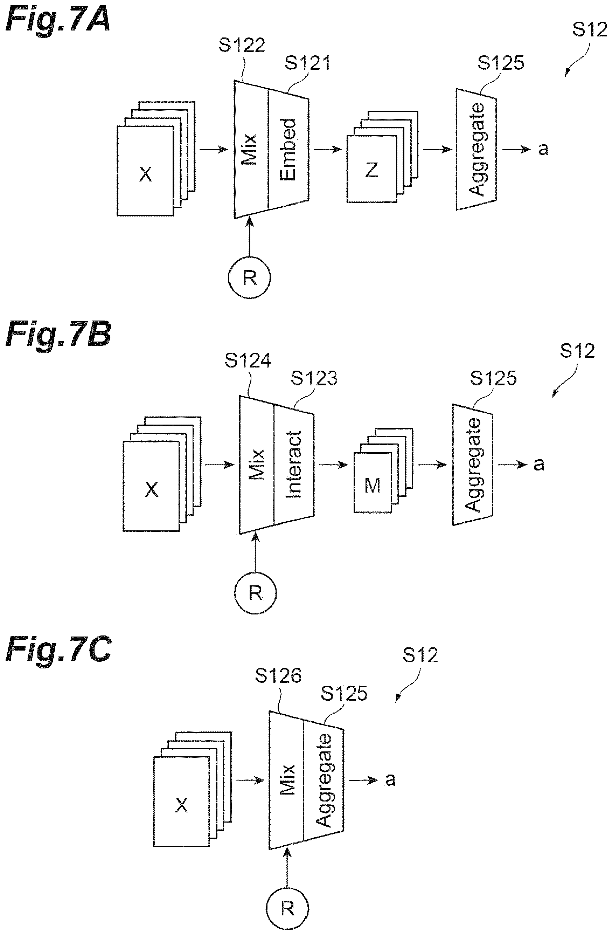

As described above, the procedure for calculating the composite feature vector is not limited. Other examples of the calculation procedure will be described with reference to FIGS. 7A, 7B and 7C. FIGS. 7A, 7B and 7C are diagrams showing other examples of details of step S12.

As shown in an example of FIG. 7A, the calculation unit 12 may calculate the composite feature vector by using the machine learning for the embedding function and the aggregation function, without using the machine learning for the interaction function. In one example, the calculation unit 12 executes steps S121, S122, and S125 to calculate the composite feature vector. In step S121, the embedding unit 121 calculates the feature vectors Z from the numerical representations X for the plurality of component objects by the machine learning for the embedding function. In step S122, the ratio application unit 124 executes the application of the combination ratios R in association with the machine learning model for the embedding function. As described above, the timing of applying the combination ratios R is not limited. In step S125, the aggregation unit 123 calculates the composite feature vector a from the plurality of feature vectors Z reflecting the plurality of combination ratios R. In one example, the aggregation unit 123 inputs a set of the plurality of feature vectors Z into the machine learning model for the aggregation function to calculate the composite feature vector a. Alternatively, the aggregation unit 123 may input the set of the plurality of feature vectors Z to an aggregation function that does not use the machine learning to calculate the composite feature vector a.

In the example of FIG. 7A, since the machine learning for the embedding function is included, even in a case where the numerical representation X is unstructured data that is data not expressed by a fixed-length vector, the feature vector Z that is a fixed-length vector can be generated from such numerical representation X. Such processing is referred to as feature learning. By the feature learning, it is possible to reduce domain knowledge necessary for construction of a machine learning model (learned model) and improve prediction accuracy.

As shown in an example of FIG. 7B, the calculation unit 12 may calculate the composite feature vector by the machine learning for the interaction function and the aggregation function, without using the machine learning for the embedding function. In one example, the calculation unit 12 executes steps S123, S124, and S125 to calculate the composite feature vector. In step S123, the interacting unit 122 calculates the feature vectors M from the numerical representations X for the plurality of component objects by the machine learning for the interaction function. In step S124, the ratio application unit 124 executes the application of the combination ratios R in association with the machine learning model for the interaction function. As described above, the timing of applying the combination ratios R is not limited. In step S125, the aggregation unit 123 calculates the composite feature vector a from the plurality of feature vectors M reflecting the plurality of combination ratios R. In one example, the aggregation unit 123 inputs a set of the plurality of feature vectors M into the machine learning model for the aggregation function to calculate the composite feature vector a. Alternatively, the aggregation unit 123 may input a set of the plurality of feature vectors M to an aggregation function that does not use the machine learning to calculate the composite feature vector a.

In the example of FIG. 7B, since the machine learning for the interaction function is included, it is possible to accurately learn a nonlinear response caused by the change in the combination of the numerical representations X.

As shown in an example of FIG. 7C, the calculation unit 12 may calculate the composite feature vector by the machine learning for the aggregation function, without using the machine learning for the embedding function and the machine learning for the interaction function. In one example, the calculation unit 12 executes steps S125 and S126 to calculate the composite feature vector. In step S125, the aggregation unit 123 inputs a set of the plurality of numerical representations X corresponding to the plurality of component objects into the machine learning model for the aggregation function to calculate the composite feature vector a. In step S126, the ratio application unit 124 executes the application of the combination ratios R in association with the machine learning model for the aggregation function. As described above, the timing of applying the combination ratios R is not limited.

In the example of FIG. 7C, since the processing procedure for calculating the composite feature vector is simple, the calculation load can be reduced.

As described above, various methods can be considered as a procedure for obtaining the composite feature vector a from the plurality of numerical representations X. In any case, the calculation unit 12 executes each of the machine learning and the application of the combination ratios at least once to calculate the composite feature vector.

At least one of the embedding unit 121 and the interacting unit 122, the aggregation unit 123, and the ratio application unit 124 may be constructed by one neural network. That is, the calculation unit 12 may be constructed by one neural network. In other words, all the embedding unit 121, the interacting unit 122, the aggregation unit 123, and the ratio application unit 124 are part of the of the single neural network. In a case where such a single neural network is used, the ratio application unit 124 applies the ratios in the intermediate layer, as shown in FIG. 5.

### [Program]

An information processing program for causing a computer or a computer system to function as the information processing system 10 includes a program code for causing the computer system to function as the acquisition unit 11, the calculation unit 12 (the embedding unit 121, the interacting unit 122, the aggregation unit 123, and the ratio application unit 124), and the prediction unit 13. The information processing program may be provided after being non-temporarily recorded on a tangible recording medium such as a CD-ROM, a DVD-ROM, or a semiconductor memory. Alternatively, the information processing program may be provided through a communication network as a data signal superimposed on a carrier wave. The provided information processing program is stored in, for example, the auxiliary storage unit 103. Each of the functional elements described above is realized by the processor 101 reading the information processing program from the auxiliary storage unit 103 and executing the information processing program.

### [Effect]

As described above, an information processing system according to one aspect of the present disclosure includes at least one processor. The at least one processor is configured to: acquire a numerical representation and a combination ratio for each of a plurality of component objects; execute, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and output the composite feature vector.

An information processing method according to one aspect of the present disclosure is executed by an information processing system including at least one processor. The information processing method includes: acquiring a numerical representation and a combination ratio for each of a plurality of component objects; executing, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and outputting the composite feature vector.

An information processing program according to one aspect of the present disclosure causes a computer to execute: acquiring a numerical representation and a combination ratio for each of a plurality of component objects; executing, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and outputting the composite feature vector.

In such an aspect, since the machine learning and the application of the combination ratios are executed for each component object, it is possible to improve the accuracy of analysis of composite object even in a case where a sufficient amount of data cannot be prepared for the component objects.

In the information processing system according to another aspect, the at least one processor may be configured to: input the plurality of numerical representations into a machine learning model to calculate a feature vector of each of the plurality of component objects; execute the application of the plurality of combination ratios in association with the machine learning model; and input a plurality of the feature vectors reflecting the plurality of combination ratios into an aggregation function to calculate the composite feature vector. This series of procedures makes it possible to increase the accuracy of analysis of the composite object even in a case where a sufficient amount of data cannot be prepared for the component object.

In the information processing system according to another aspect, the at least one processor may be further configured to: input the plurality of numerical representations into a first machine learning model to calculate a first feature vector of each of the plurality of component objects; input a plurality of the first feature vectors into a second machine learning model to calculate a second feature vector of each of the plurality of component objects; execute the application of the plurality of combination ratios in association with at least one machine learning model selected from the first machine learning model and the second machine learning model; and input a plurality of the second feature vectors reflecting the plurality of combination ratios into an aggregation function to calculate the composite feature vector. By performing the machine learning in two stages, it is possible to further increase the accuracy of analysis of the composite object even in a case where a sufficient amount of data cannot be prepared for the component object.

In the information processing system according to another aspect, the first machine learning model may be a machine learning model which generates the first feature vector that is a fixed-length vector from the numerical representation that is unstructured data. By using the first machine learning model, the composite feature vector can be obtained from a numerical representation that cannot be expressed by a fixed-length vector.

In the information processing system according to another aspect, the application of the plurality of combination ratios in association with the machine learning model may include applying the plurality of combination ratios to output data of an intermediate layer of the machine learning model. By setting the timing of applying the combination ratios in this manner, the combination ratios can be appropriately applied regardless of whether the data input to the machine learning model is unstructured or structured.

In the information processing system according to another aspect, the at least one processor may be further configured to: input the composite feature vector into another machine learning model to calculate a predicted value of characteristics of the composite object; and output the predicted value. By this processing, it is possible to accurately calculate the characteristics of the composite object.

In the information processing system according to another aspect, the component object may be a material, and the composite object may be a multi-component substance. In this case, it is possible to increase the accuracy of analysis of the multi-component substance even in a case where a sufficient amount of data for the material cannot be prepared.

In the information processing system according to another aspect, the material may be a polymer, and the multi-component substance may be a polymer alloy. In this case, it is possible to increase the accuracy of analysis of the polymer alloy even in a case where a sufficient amount of data for the polymer cannot be prepared. There are a huge variety of polymer alloys, and correspondingly, there are a huge variety of polymers. For such polymers and polymer alloys, in general, only some of the possible combinations can be tested, and thus a sufficient amount of data cannot be obtained in many cases. According to this aspect, it is possible to accurately analyze the polymer alloy even in a case where the amount of data is not sufficient as described above.

### [Modifications]

The present invention has been described in detail based on the embodiment. However, the present invention is not limited to the embodiment described above. The present invention can be modified in various ways without departing from its gist.

In the embodiment described above, the information processing system 10 includes the prediction unit 13, but this functional element may be omitted. That is, the process of predicting the characteristics of the composite object may be performed by a computer system different from the information processing system.

The processing procedure of the information processing method executed by at least one processor is not limited to the example in the embodiment described above. For example, some of the steps (processes) described above may be omitted, or the steps may be executed in a different order. In addition, any two or more steps among the above-described steps may be combined, or a part of each step may be modified or deleted. Alternatively, other steps may be executed in addition to each of the above steps. For example, the processing of steps S14 and S15 may be omitted. In step S12 shown in FIG. 4, any one or two of steps S122, S124, and S126 may be omitted.

In a case of comparing the magnitudes of two numerical values in the information processing system, either of the two criteria of "equal to or greater than" and "greater than" may be used, or either of the two criteria of "equal to or less than" and "less than" may be used. Such criteria selection does not change the technical significance of the process of comparing the magnitudes of two numerical values.

In the present disclosure, the expression "at least one processor performs a first process, performs a second process, ..., and performs an n-th process" or the expression corresponding thereto shows a concept including a case where an execution subject (that is, a processor) of n processes from the first process to the n-th process changes on the way. That is, this expression shows a concept including both a case where all of the n processes are performed by the same processor and a case where the processor is changed according to an any policy in the n processes.

### Reference Signs List

10: information processing system, 11: acquisition unit, 12: calculation unit, 13: prediction unit, 121: embedding unit, 122: interacting unit, 123: aggregation unit, 124: ratio application unit.

## Claims

1. An information processing system comprising:
at least one processor,
wherein the at least one processor is configured to:
acquire a numerical representation and a combination ratio for each of a plurality of component objects;
execute, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and
output the composite feature vector.

2. The information processing system according to claim 1, wherein the at least one processor is configured to:
input the plurality of numerical representations into a machine learning model to calculate a feature vector of each of the plurality of component objects;
execute the application of the plurality of combination ratios in association with the machine learning model; and
input a plurality of the feature vectors reflecting the plurality of combination ratios into an aggregation function to calculate the composite feature vector.

3. The information processing system according to claim 1, wherein the at least one processor is configured to:
input the plurality of numerical representations into a first machine learning model to calculate a first feature vector of each of the plurality of component objects;
input a plurality of the first feature vectors into a second machine learning model to calculate a second feature vector of each of the plurality of component objects;
execute the application of the plurality of combination ratios in association with at least one machine learning model selected from the first machine learning model and the second machine learning model; and
input a plurality of the second feature vectors reflecting the plurality of combination ratios into an aggregation function to calculate the composite feature vector.

4. The information processing system according to claim 3,
wherein the first machine learning model is a machine learning model which generates the first feature vector that is a fixed-length vector from the numerical representation that is unstructured data.

5. The information processing system according to any one of claims 2 to 4,
wherein the application of the plurality of combination ratios in association with the machine learning model comprises applying the plurality of combination ratios to output data of an intermediate layer of the machine learning model.

6. The information processing system according to any one of claims 1 to 5, wherein the at least one processor is further configured to:
input the composite feature vector into another machine learning model to calculate a predicted value of characteristics of the composite object; and
output the predicted value.

7. The information processing system according to any one of claims 1 to 6,
wherein the component object is a material, and the composite object is a multi-component substance.

8. The information processing system according to claim 7,
wherein the material is a polymer, and the multi-component substance is a polymer alloy.

9. An information processing method executable by an information processing system including at least one processor, the method comprising:
acquiring a numerical representation and a combination ratio for each of a plurality of component objects;
executing, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and
outputting the composite feature vector.

10. An information processing program causing a computer to execute:
acquiring a numerical representation and a combination ratio for each of a plurality of component objects;
executing, based on a plurality of the numerical representations and a plurality of the combination ratios corresponding to the plurality of component objects, machine learning and application of the plurality of combination ratios to calculate a composite feature vector indicating features of a composite object obtained by combining the plurality of component objects; and
outputting the composite feature vector.
